**Europäisches Patentamt**

**European Patent Office**

**Office européen des brevets**

(11) Veröffentlichungsnummer: **0 263 948 B1**

## (12) EUROPÄISCHE PATENTSCHRIFT

(45) Veröffentlichungstag der Patentschrift: **12.02.92**

(21) Anmeldenummer: **87112398.0**

(22) Anmeldetag: **26.08.87**

(51) Int. Cl.⁵: **C12M 1/40**, G01N 33/48

---

(54) **Biosensor.**

---

(30) Priorität: **10.10.86 DE 3634573**

(43) Veröffentlichungstag der Anmeldung:
**20.04.88 Patentblatt 88/16**

(45) Bekanntmachung des Hinweises auf die
Patenterteilung:
**12.02.92 Patentblatt 92/07**

(84) Benannte Vertragsstaaten:
**CH DE FR GB IT LI**

(56) Entgegenhaltungen:
**EP-A- 0 193 882**

**BIOTECH.' 83, PROCEEDINGS OF THE INTER-
NATIONAL CONFERENCE ON THE COMMER-
CIAL APPLICATIONS OF BIOTECHNOLOGY,
Londen, 4.-6. Mai 1983, Seiten 665-678, Online Conf. Ltd, Northwood, GB; K. MOSBACH
et al.: "New biosensor devices"**

**JOURNAL OF PHYSICS E. SCIENTIFIC IN-
STRUMENTS, Band 18, Nr. 9, September
1985, Seiten 736-749, The Institute of Physics, Bristol, GB; C. NYLANDER: "Chemical
and biological sensors"**

(73) Patentinhaber: **Messerschmitt-Bölkow-Blohm
Gesellschaft mit beschränkter Haftung
Robert-Koch-Strasse
W-8012 Ottobrunn(DE)**

(72) Erfinder: **Hilpert, Reinhold, Dr.
Marbachstrasse 15a
W-8000 München 70(DE)**

---

## Beschreibung

Die Erfindung betrifft einen Biosensor zur Detektion von Schwermetallen mit einer biologischen Komponente, welche auf einem Transducer immobilisiert ist.

Biosensoren sind Meßfühler wie sie beispielsweise in "Bild der Wissenschaft", Heft 4-1986, S. 100 - 110 beschrieben sind und bei denen eine biologische Komponente primär mit dem zu bestimmenden Parameter reagiert. Diese Primärreaktion oder ein Produkt dieser Primärreaktion wird von einem sog. Transducer erfaßt und in ein verwertbares, im allgemeinen elektrisches Meßsignal überführt. Dazu ist es erforderlich, daß die biologische Komponente in enger räumlicher Nachbarschaft mit dem Transducer immobilisiert, d.h. fixiert, wird. Immobilisierungsmethoden sind z.B. die Adsorption an einem anorganischen oder organischen Träger, der physikalische Einschluß in Gel-Matrizes oder hinter einer semipermeablen Membran, die Quervernetzung durch bifunktionelle oder multifunktionelle Reagentien und kovalente Bindung an einen Transducer, welche entweder direkt oder unter Zuhilfenahme von sog. "Linkern" erfolgen kann.

Dauerhaft funktionierende Biosensoren sind zwar noch nicht auf dem Markt, jedoch lassen sich Anwendungen in der medizinischen Analytik, Abwasser-Überwachung oder in Bioreaktoren absehen. Die vorliegende Erfindung hat sich zur Aufgabe gesetzt, einen Biosensor zu schaffen, der zur Detektion von Schwermetallen geeignet ist, und der damit die bisher gebräuchlichen analytischen Methoden, z.B. die Sulfidfällung, zur Erkennung von Schwermetallen ersetzen kann.

Diese Aufgabe wird durch einen nach den kennzeichnenden Merkmalen des Patentanspruchs 1 ausgebildeten Biosensor gelöst.

Die Erfindung macht sich die besondere Eigenschaft der Phytochelatine (PC) zunutze, Schwermetallionen wie $CD^{2+}$, $Hg^{2+}$, $Zn^{2+}$, $Pb^{2+}$ oder $Cu^{2+}$ zu binden. Diese Eigenschaft wurde von Grill, Winnakker und Zenk in "SCIENCE", 08. November 1985, Vol. 230, S. 674 bis 676 erstmals beschrieben. PC sind pflanzliche Peptide, die aus den Aminosäuren Glycin, Cystein und Glutaminsäure aufgebaut sind. Die allgemeine Strukturformel lautet:

($\gamma$-glu-cys)$_n$-gly,

wobei n i.A. zwischen 3 und 8 variiert. Ihre biologische Aufgabe besteht in der Entgiftung pflanzlicher Gewebe, wobei die Anwesenheit von Schwermetallen in der Pflanze die Bildung von PC induziert. PC können daher aus Pflanzenzellenkulturen verschiedener Spezies isoliert werden (sh. obengenannter Artikel aus SCIENCE). Die Bindung der Schwermetalle an PC ist formal als Metallmercaptid zwischen den SH-Gruppen des Cysteins und den Schwermetallionen aufzufassen. Da die PC ausschließlich Schwermetalle binden, also nicht beispielsweise Alkali- oder Erdalkali-Metalle, ist es möglich, diese PC als biologische Komponente für einen Biosensor zur Detektion von Schwermetallen zu verwenden.

Die Erfindung soll im folgenden anhand einiger Ausführungsbeispiele, die in den Figuren teilweise schematisch dargestellt sind, näher beschrieben werden.

Es zeigen:

Fig. 1    den prinzipiellen Aufbau einer Meßeinrichtung mit einem Biosensor;

Fig. 2    einen Biosensor mit einem als Feldeffekttransistor ausgebildeten Transducer;

Fig. 3    eine Schaltung mit einem Biosensor gemäß Fig. 2;

Fig.4    einen Biosensor mit einem als Piezokristall ausgebildeten Transducer;

Fig. 5    einen Biosensor mit einem als Lichtleiter ausgebildeten Transducer;

Fig. 6    einen Biosensor mit einem Transducer zur Messung der Leitfähigkeit bzw. der Dielektrizitätskonstanten.

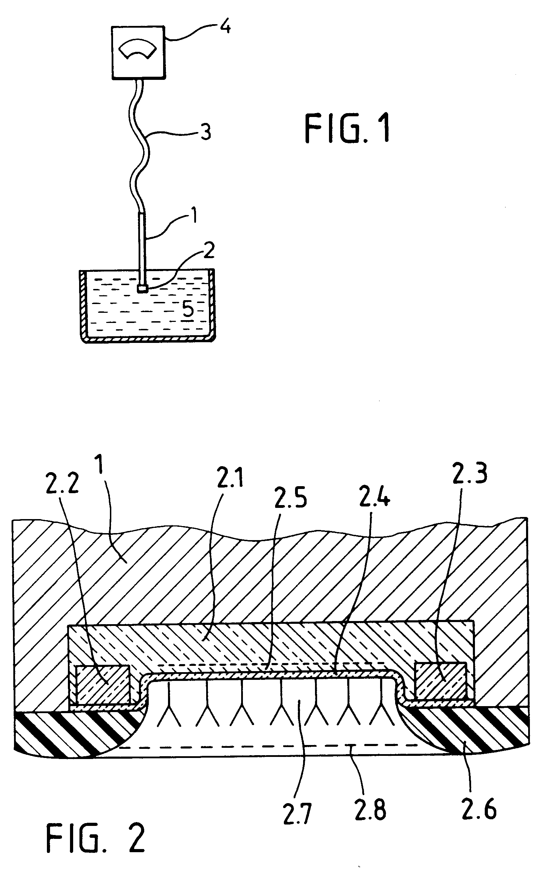

Die in Fig. 1 schematisch dargestellte Meßanordnung weist eine Sensorträger 1 auf, an dessen Spitze der eigentliche Biosensor 2 angeordnet ist, dessen Ausgangssignale über eine Übertragungsleitung 3 einer Verstärker- und Anzeigeeinrichtung 4 zugeführt werden. Der Biosensor 2 wird in das Meßsubstrat 5 eingetaucht, welches gasförmig, flüssig oder fest sein kann, und welches bei Vorhandensein eines bestimmten zu messenden Parameters mit der biologischen Komponente des Biosensors reagiert.

Fig. 2 zeigt ein Ausführungsbeispiel für einen Biosensor, bei dem der Transducer als Feldeffekttransistor aufgebaut ist. In einer stirnseitigen Ausnehmung des Sensorträgers 1 ist dabei ein Siliciumblock 2.1 eingelassen, in dem in an sich bekannter Weise eine Source-Elektrode 2.2 und eine Drain-Elektrode 2.3 durch entsprechende Dotierung erzeugt wird. Die Source- und Drain-Elektrode 2.2 und 2.3 sowie die dazwischen liegende Siliciumschicht, die den Leitkanal 2.5 bildet, werden mit einer Isolierschicht 2.4, beispielsweise aus $Si_3N_4$, abgedeckt. Die Randbereiche des Siliciumblocks 2.1 bzw. der Isolierschicht 2.4 sowie des angrenzenden Sensorträgers werden zusätzlich noch mit einer Vergußmasse 2.6 abgedeckt. Als biologische Komponente des Biosensors werden Phytochelatine (PC) auf der Isolierschicht 2.4 immobilisiert, welch damit das Gate 2.7 des Feldeffekttransistors bilden. Hierzu darf die eigentliche Gate-Elektrode, die typischerweise bei Feldeffekttransistoren vorhanden ist, nicht als solche ausgeführt sein. Als Immobilisierungsreagentien können beispielsweise Glutardialdehyd und Serumalbumin verwendet werden (vgl. L.D. Bowers und P.W. Carr "Immobilized Enzymes in Analytical Chemistry").

Durch Einlagerung von Schwermetallionen in die PC baut sich in der als Gate wirkenden PC-Schicht 2.7 ein elektrisches Feld auf, welches im Leitkanal 2.5 Ladungsträger induziert und somit den Widerstand des Leitkanals 2.5 ändert. Zur Einstellung eines optimalen Arbeitspunktes des Feldeffekttransistors ist die PC-Schicht 2.7 mit einer zusätzlichen gitterförmigen Gate-Elektrode 2.8 versehen, über die eine externe Vorspannung angelegt werden kann und deren Gitterabstände so gewählt sind, daß Reaktionen des Meßsubstrats mit der PC-Schicht nicht behindert werden.

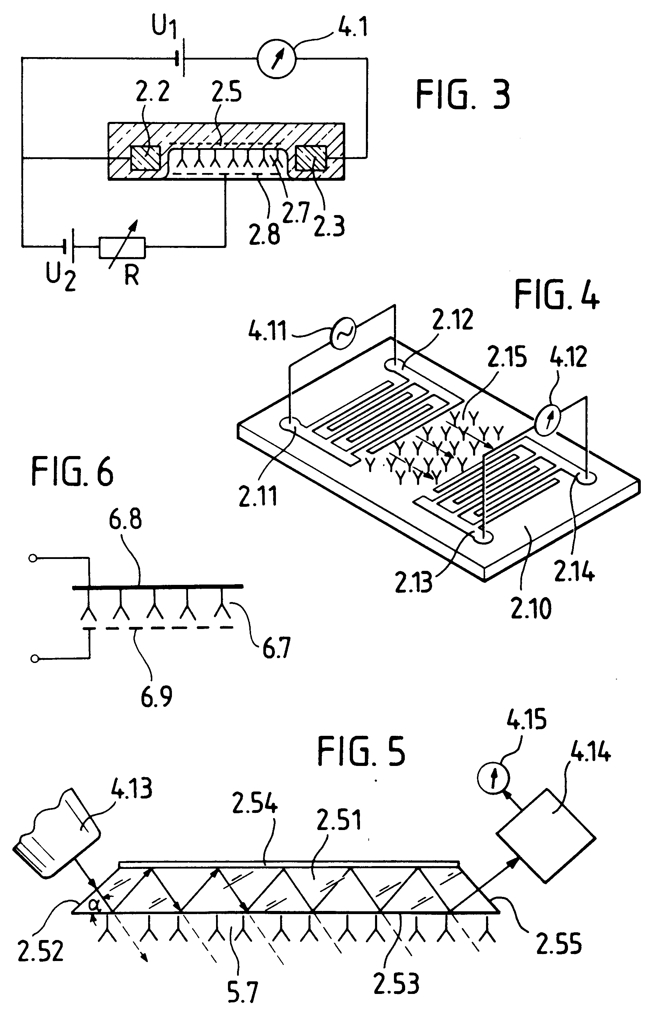

Eine entsprechende Schaltung zum Betrieb des in Fig. 2 dargestellten, als Feldeffekttransistor ausgebildeten Biosensors ist in Fig. 3 dargestellt. Dabei ist die Source-Elektrode 2.2 und die Drain-Elektrode 2.3 mit einer Spannungsquelle $U_1$ verbunden, wobei der durch den Leitkanal 2.5 fließende Strom mit einem geeigneten Meßgerät 4.1 gemessen wird. Über die zusätzliche Gate-Elektrode 2.8 kann eine über ein Potentiometer R regelbare Vorspannung aus einer Spannungsquelle $U_2$ auf den Leitkanal 2.5 einwirken, wodurch eine Einstellung des Arbeitsbereiches des Feldeffekttransistors erzielt wird.

Da der Schwermetallsensor auf der Basis von PC primär nicht reversibel ist, wird durch die zusätzliche Gate-Elektrode 2.8 noch die Möglichkeit gegeben, durch entsprechende Polung der Spannungsquelle $U_2$ die Schwermetallionen wieder aus den PC herauszulösen. Eine andere Möglichkeit zur Regenerierung des Biosensors besteht in der Spülung mit Säuren, durch welche die Schwermetallionen aus dem PC-Schwermetall-Komplex herausgelöst werden.

Da die PC unter oxidierenden Bedingungen nur stabil sind, wenn sie als $PC^{2-}$-Metall$^{2+}$-Komplex vorliegen, empfiehlt es sich, diese durch Komplexierung mit einem nur schwach bindenden Metall, wie z.B. Eisen, zu stabilisieren.

Fig. 4 zeigt den prinzipiellen Aufbau eines Biosensors zur Detektion von Schwermetallen auf der Basis eines Oberflächenwellen-Bauelementes. Hierzu sind auf einer Oberfläche eines piezoelektrischen Substrats 2.10 aus Lithium-Niobat kammartige Elektroden 2.11 und 2.12 bzw. 2.13 und 2.14 angeordnet. Die Elektroden 2.11 und 2.12 sind mit einer Wechselspannungsquelle 4.11 verbunden. Die Frequenz der Wechselspannungsquelle 4.11 sowie die Finger der Elektroden 2.11 und 2.12 sind so ausgelegt, daß jede erzeugte und weitergeleitete Oberflächenwelle die nächste verstärkt, so daß sich auf der Oberfläche des Kristalls 2.10 große Amplitudenwerte ergeben. Die Rückwandlung der so erzeugten und in der Figur durch Pfeile angedeuteten Oberflächenwellen geschieht über die Elektroden 2.13 und 2.14, welch die gleiche oder eine ähnliche Geometrie wie die Elektroden 2.11 und 2.12 aufweisen. Das empfangene Signal wird durch ein Meßgerät 4.12 angezeigt.

Zwischen den Elektrodenpaaren 2.11, 2.12 und 2.13, 2.14 ist auf der Oberfläche des piezoelektrischen Substrats 2.10 eine PC-Schicht 2.15 immobilisiert. Durch Bindung von Schwermetallionen in dieser PC-Schicht 2.15 wird ein äußeres elektrisches Feld erzeugt, welches die Amplitude der akustischen Oberflächenwelle beeinflußt, indem es in Wechselwirkung mit dem elektrischen Feld der Oberflächenwelle tritt. Die entsprechende Änderung im Ausgangssignal wird durch das Meßgerät 4.12 angezeigt.

In analoger Weise zur Ausführungsform gemäß Fig. 4 kann die PC-Schicht auch auf der Oberfläche eines piezoelektrischen Kristalls immobilisiert sein, der mittels eines elektrischen Schwingkreises in seiner Eigenfrequenz erregt wird. Die Verschiebung bzw. Schwächung der Resonanzschwingung durch Einlagerung von Schwermetallen in die PC-Schicht wird dann gemessen.

Fig.5 zeigt einen Biosensor zur Detektion von Schwermetallen, bei dem als Transducer ein prismatischer Lichtleiter 2.51 verwendet wird. Das von einer Lichtquelle 4.13 ausgesandte Licht tritt auf der Stirnseite 2.52 des Lichtleiters 2.51 ein und wird an den Flächen 2.53 und 2.54 mehrfach reflektiert, bis es an der gegenüberliegenden Stirnfläche 2.55 austritt und dort von einem optoelektrischen Empfänger 4.14 registriert wird. Die Fläche 2.54 ist verspiegelt, wohingehend auf der Fläche 2.53 eine PC-Schicht 5.7 immobilisiert ist. Änderungen des Brechungsindizes der PC-Schicht 5.7 durch Aufnahme von Schwermetallionen bewirken daher Änderungen der an dieser Grenzfläche nicht reflektierten und somit austretenden Lichtintensität und damit auch eine Änderung der im Empfänger 4.14 empfangenen Intensität. Diese Änderung wird in einem Meßgerät 4.15 angezeigt. Der Einfallswinkel $\alpha$ des Lichtes an den Flächen 2.53 bzw. 2.54 des Lichtleiters sollte einerseits möglichst klein gewählt werde, andererseits sollte eine ausreichende Anzahl von Reflexionen stattfinden.

Eine weitere Ausführungsform eines Biosensors zur Detektion von Schwermetallen besteht in der in Fig. 6 schematisch dargestellten Anordnung, bei der eine PC-Schicht 6.7 auf einer ersten Elektrode 6.8 immobilisiert ist und mit einer zweiten, gitterförmigen Elektrode 6.9 abgedeckt ist. Beide Elektroden 6.8, 6.9 sind mit einer an sich bekannten und deshalb nicht dargestellten Meßeinrichtung zur Bestimmung der Leitfähigkeit, welche sich durch Einlagerung von Schwermetallionen in die PC-Schicht 6.7 ändert, verbunden.

Eine ähnliche Anordnung kann verwendet werden, wenn als Meßgröße die Änderung der Dielektrizitätskonstanten der PC-Schicht 6.7 herangezogen werden soll. Hierbei ist es vorteilhaft, mindestens eine der Elektroden 6.8 bzw. 6.9 durch eine dünne Isolierschicht galvanisch von der PC-Schicht 6.7 zu trennen. Die Anordnung ist dann in eine an sich bekannte Wechselstrommeßbrücke als variable Kapazität geschaltet.

## Patentansprüche

1. Biosensor zur Detektion von Schwermetallen mit einer biologischen Komponente, welche auf einem Transducer immobilisiert ist, dadurch **gekennzeichnet,** daß als biologische Komponente mindestens ein Peptid aus der Gruppe der Phytochelatine verwendet wird.

2. Biosensor nach Anspruch 1, dadurch **gekennzeichnet,** daß der Transducer als Feldeffekttransistor ausgebildet ist und, daß das bzw. die Phytochelatin(e) (2.7) schichtförmig am Gate immobilisiert wird bzw. werden.

3. Biosensor nach Anspruch 2, dadurch **gekennzeichnet,** daß die Phytochelatin(e)-Schicht (2.7) mit einer zusätzlichen Gate-Elektrode (2.8) versehen ist.

4. Biosensor nach einem der Ansprüche 1 bis 3, dadurch **gekennzeichnet,** daß der Transducer einen piezoelektrischen Kristall (2.10) aufweist, an dessen Oberfläche das bzw. die Phytochelatin(e) (2.15) schichtförmig immobilisiert wird bzw. werden.

5. Biosensor nach einem der Ansprüche 1 bis 4, dadurch **gekennzeichnet,** daß der Transducer einen Lichtleiter (2.51) aufweist, wobei auf mindestens einer Oberfläche (2.53) des Lichtleiters (2.51) das bzw. die Phytochelatin(e) (5.7) schichtförmig immobilisiert wird bzw. werden, derart, daß die Bindung von Schwermetallen in der Phytochelatin(e)-Schicht eine Änderung der optischen Eigenschaften des Lichtleiters (2.51) bewirkt.

6. Biosensor nach einem der Ansprüche 1 bis 5, dadurch **gekennzeichnet,** daß das, bzw. die Phytochelatin(e) (6.7) zwischen zwei Elektroden (6.8, 6.9) angeordnet ist, welche mit einer Einrichtung zur Messung der Leitfähigkeit oder der Dielektrizitätskonstanten verbunden sind.

## Claims

1. A biosensor for detecting heavy metals with a biological component which is immobilised on a transducer, characterised in that at least one peptide from the group of the phytochelatines is used as the biological component.

2. A biosensor according to claim 1, characterised in that the transducer is designed as a field effect transistor and in that the phytochelatine(s) (2.7) is or are immobilised in a layer-shaped manner at the gate.

3. A biosensor according to claim 2, characterised in that the layer of phytochelatine(s) is provided with an additional gate electrode (2.8).

4. A biosensor according to one of claims 1 to 3, characterised in that the transducer has a piezoelectric crystal (2.10), on the surface of which the phytochelatine(s) (2.15) is or are immobilised in a layer-shaped manner.

5. A biosensor according to one of claims 1 to 4, characterised in that the transducer (2.51) has a photoconductor (2.51), in which respect the phytochelatine(s) (5.7) is or are immobilised in a layer-shaped manner on at least one surface (2.53) of the photoconductor (2.51), in such a way that the bonding of heavy metals in the layer of phytochelatine(s) brings about a change in the optical properties of the photoconductor (2.51)

6. A biosensor according to one of claims 1 to 5, characterised in that the phytochelatine(s) (6.7) is arranged between two electrodes (6.8, 6.9), which are connected to a mechanism for measuring the conductivity or the dielectric constant.

## Revendications

1. Biocapteur pour la détection de métaux lourds comportant un composant biologique qui est immobilisé sur un transducteur, caractérisé en ce qu'on utilise comme composant biologique au moins un peptide du groupe des phytochélatines.

2. Biocapteur selon la revendication 1, caractérisé en ce que le transducteur est réalisé sous la forme d'un transistor à effet de champ et en ce que la ou les phytochélatine(s) (2.7) est ou sont immobilisée(s) en couche sur la grille.

3. Biocapteur selon la revendication 2, caractérisé en ce que la couche de phytochélatine(s) (2.7) est munie d'une grille supplémentaire (2.8).

4.  Biocapteur selon l'une des revendications 1 à 3, caractérisé en ce que le transducteur présente un cristal piézo-électrique (2.10) sur la surface duquel la ou les phytochélatine(s)(2.15) est ou sont immobilisée(s) en couche.

5.  Biocapteur selon l'une des revendications 1 à 4, caractérisé en ce que le transducteur présente une fibre optique (2.51), la ou les phytochélatine(s) (2.7) étant immobilisée(s) en couche sur au moins une surface (2.53) de la fibre optique (2.51) de manière telle que la liaison de métaux lourds dans la couche de phytochélatine(s) provoque une modification des caractéristiques optiques de la fibre optique (2.51).

6.  Biocapteur selon l'une des revendications 1 à 5, caractérisé en ce que la ou les phytochélatine(s) (6.7) est (sont) disposée(s) entre deux électrodes qui sont reliées à un dispositif pour la mesure de la conductibilité ou de la constante diélectrique.

FIG. 1

FIG. 2

FIG. 3

FIG. 4

FIG. 6

FIG. 5